# EUROPÄISCHE PATENTANMELDUNG

(11) **EP 0 526 758 A1**
(43) Veröffentlichungstag der Anmeldung: **10.02.1993**
(21) Anmeldenummer: 92111705.7
(22) Anmeldetag: 09.07.1992
(51) Int. Cl.: A61B 17/22, A61B 8/00

(54) **Lithotripter mit Ankopplungsdetektor**

(30) Priorität: 06.08.1991 DE 4125950
(71) Anmelder: DORNIER MEDIZINTECHNIK GMBH, D-82101 Germering (DE)
(72) Erfinder: Warnking, Reinhard, Dipl.-Ing., Tempe, Arizona 85283 (US); Grünwald, Sorin, Dipl.-Ing., W-5650 Solingen 19 (DE)
(74) Vertreter: Landsmann, Ralf, Dipl.-Ing.

(57) **Zusammenfassung**

Die Erfindung betrifft ein Verfahren zur Detektion der Güte der Ankopplung eines Stoßwellentherapiekopfs (TK) an den Patientenkörper (PK). Dazu wird ein Vergleich zwischen einem Ultraschallbild (Y), aufgenommen ohne Ankopplung (Leeraufnahme) mit dem aktuellen Ultraschallbild (X), aufgenommen bei angekoppeltem Patientenkörper (PK), durchgeführt.

## Beschreibung

Die Erfindung betrifft einen Verfahren nach dem Oberbegriff des Anspruch 1.

Im Rahmen der extrakorporalen Stoßwellenlithotripsie (ESWL) wird der Patientenkörper an den Therapiekopf des Stoßwellentherapiegeräts mit einem Wasserkissen angekoppelt. Bei ungenügender Ankopplung werden Stoßwellen an Wasser-Luft- Übergangsflächen zurück in den Therapiekopf reflektiert. Dies hat eine Beschädigung des im Therapiekopfs angeordneten In-Line-Schallkopfs eines Ultraschallortungsgeräts zur Folge. Außerdem wird bei ungenügender Ankopplung der Therapieerfolg erheblich beeinträchtigt, was eine vermeidbare Patientenbelastung darstellt.

Aus der DE 39 13 023 A1 ist ein Zertrümmerungswellen-Behandlungsgerät bekannt, bei dem ein Abbildungs-Ultraschallwander im Zertrümmerungswellentherapiekopf integriert ist. Aus vor und während der Anwendung der Zertrümmerungswellen aufgenommenen Ultraschallbildern werden Subtraktionsbilder erzeugt, um den Zustand des zu zerstörenden Konkrements zu überwachen.

Aus der DE 39 00 893 A1 ist ein Stoßwellenbehandlungsgerät bekannt, bei dem die Erzeugung einer Stoßwelle verhindert wird, wenn die Überlappung des zu zerstörenden Gegenstands mit dem Brennpunkt des Stoßwellen-Therapiekopfs zu gering ist. Dazu wird die Überlappung des in einem Ultraschallbild abgebildeten zu zerstörenden Gegenstands mit einem in dieses Ultraschallbild eingespielten Brennpunktzonen-Markierung ermittelt.

Der Erfindung liegt die Aufgabe zugrunde, ein Verfahren zu schaffen, mit dem die Güte der Ankopplung detektiert werden kann.

Diese Aufgabe wird mit einem Verfahren nach Anspruch 1 gelöst. Ausgestaltungen der Erfindung sowie ein Lithotripter zur Durchführung des Verfahrens sind Gegenstände weiterer Ansprüche.

Bei dem erfindungsgemäßen Verfahren wird mit einem Ultraschallortungsgerät, dessen Schallkopf als In-Line-Schallkopf im Therapiekopf des Lithotripters integriert ist, bei angekoppeltem Patientenkörper mindestens ein Ultraschallbild aufgenommen. Dieses Ultraschallbild wird verglichen mit einem Ultraschallbild, das bei nicht angekoppelten Patientenkörper aufgenommen worden ist (Leeraufnahme).

Als Vergleichskriterium zwischen den beiden Ultraschallbildern wird die für eine ungenügende bzw. nicht vorhandene Ankopplung charakteristischen Wiederholungsechos verwendet, die bei korrekter Ankopplung fehlen.

Im Falle einer fehlerhaften Ankopplung können Warnsignale erzeugt und das Stoßwellentherapiegerät abgeschaltet werden. Durch das automatische Abschalten wird verhindert, daß die Stoßwelle zurück reflektiert wird. Dadurch wird eine unnötige Patientenbelastung vermieden und die Lebensdauer sowohl des Stoßwellentherapiekopfs wie auch des integrierten Ultraschallkopfs erheblich gesteigert.

Die Stoßwellenerzeugung im Therapiekopf kann nach sämtlichen bekannten Methoden (u.a. elektromagnetische, piezokeramische und elektrohydraulische Stoßwellenerzeugung) erfolgen.

Die Erfindung wird im weiteren anhand von Figuren beschrieben. Es zeigen:
Fig. 1 das Blockschaltbild eines erfindungsgemäßen Lithotripters zur Durchführung des Verfahrens.
Fig. 2 mehrere skizzierte Ultraschallbilder des Patientenkörpers, aufgenommen bei unterschiedlicher Güte der Ankopplung

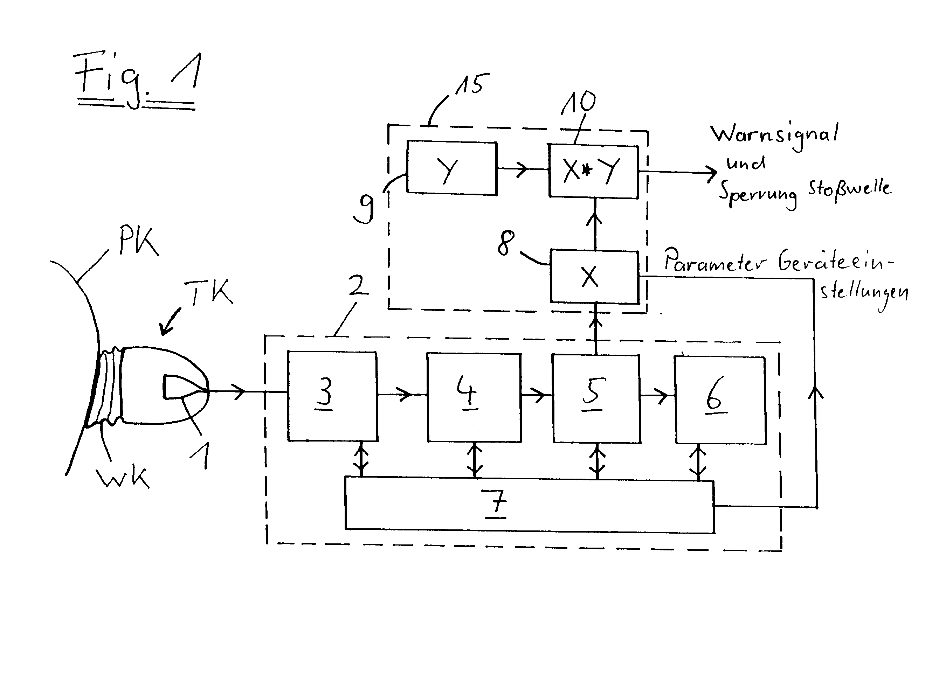

Fig. 1 zeigt das Blockschaltbild eines erfindungsgemäßen Lithotripters. Der Therapiekopf TK, in dem die Stoßwellen erzeugt werden, wird mit Hilfe eines Wasserkissens WK an den Patientenkörper PK angekoppelt. Im Therapiekopf TK ist mindestens ein Schallkopf 1 eines Ultraschallortungsgeräts 2 als In-Line-Schallkopf integriert.

Als Ultraschallortungsgerät 2 wird ein klassisches bildgebendes Ultraschalldiagnosesystem verwendet. Es besteht hier aus den Hauptbestandteilen:
- Sende-/Empfangselektronik 3 (Sende- und Empfangsansteuerung der Ultraschallwandlerelemente, Fokussierung des Schallstrahls, Steuerung des sequentiellen Bildaufbaus)
- Pulsprocessor 4 (Fiterung des Empfangssignals Demodulation und Analog/Digitalwandlung des empfangenen Signals)
- Ultraschall-Bildspeicher 5 (Mittelung der Empfangssignale zur Signal- /Rauschverbesserung und Anordnung der Empfangssignale im gewünschten Abbildungsformat)
- Display Controller 6 (Zwischenspeicherung des Bildinhalts und Umsetzung - Auslesen - im TV-Format)
- System Controller 7 (Steuerung der oben angeführten Hauptbestandteile des Ultraschallortungsgeräts 2 entsprechend den vom Benutzer gewählten Geräteeinstellungen).

Die Vorrichtung 15 zur Detektion der Güte der Ankopplung (Ankopplungsdetektor) umfaßt:
- einen Multiplizierer 8,
- einen Korrelator 10.
- einen Ultraschallbildspeicher 9.

Nach der Aufnahme eines Ultraschallbildes mit dem Ultraschallortungsgerät 2 wird der Bildinhalt (Ultraschallframe) aus dem Bildspeicher 5 ausgelesen. Die Amplitudendaten dieses Ultraschallframes (Pixel) werden mit den Parametern der Geräteeinstellungen des Ultraschallortungsgeräts 2, insbesondere Greyscale, Zoom und Power. gewichtet. Diese Wichtung wird im Multiplizierer 8 vorgenommen und dient zum Ausgleich von unterschiedlichen Geräteeinstellungen. Der gewichtete Bildinhalt X wird mit einem im Bildspeicher 9 abgelegten Vergleichsbild Y verglichen. Bei dem im Bildspeicher 9 abgelegten Vergleichsbild Y handelt es sich um eine Leeraufnahme ohne angekoppelten Patientenkörper PK.

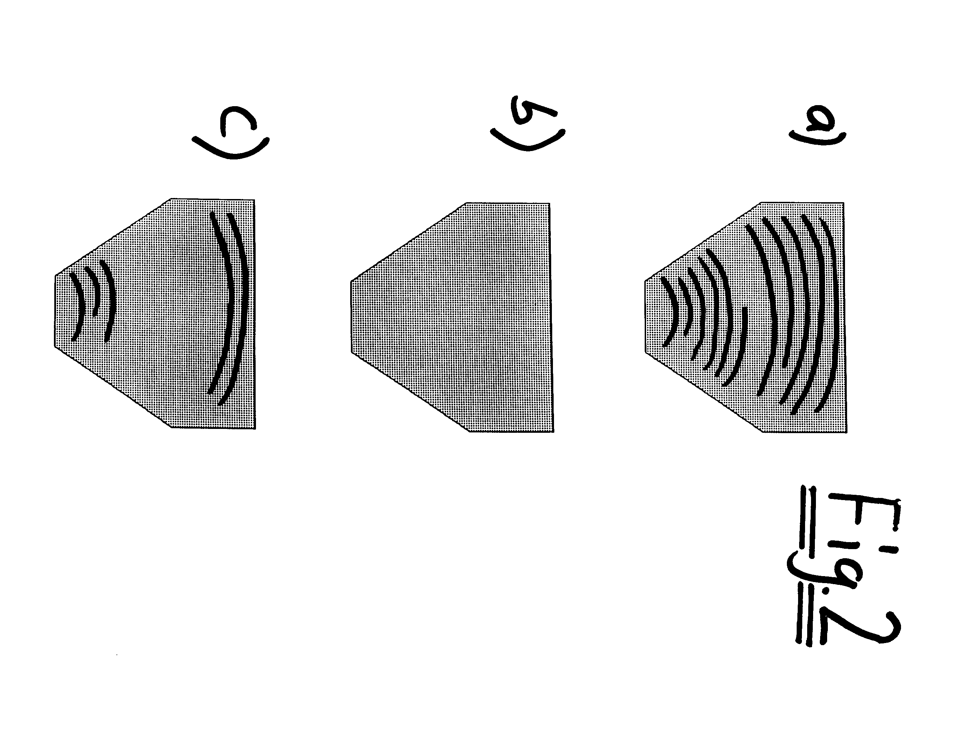

Beispiel für eine solche Leeraufnahme Y ist in Fig. 2a gezeigt. Sie zeigt charakteristische Wiederholungsechos, im Bild als Streifen zu erkennen, die bei einem korrekt angekoppeltem Patienten fehlen (Fig. 2b). Abb. 2c zeigt den Fall einer nur teilweisen, für den Therapieerfolg ungenügenden Patientenankopplung. Sie weist ebenfalls Wiederholungsechos auf, die jedoch hier weniger stark ausgeprägt sind als in Fig. 2a.

Im Korrelator 10 wird die Leeraufnahme Y aus dem Bildspeicher 9 mit dem aktuellen Ultraschallbild X, welches mit den Geräteeinstellparametern Greyscale, Zoom, Power gewichtet ist, mit Hilfe statistischer Verfahren, verglichen, wobei räumlich, d. h. über mehrere Pixel, integriert werden kann.

Tritt im aktuellen Bild X ein Wiederholungsmuster mit weitgehender Ähnlichkeit mit dem in der abgespeicherten Leeraufnahme Y vorhandenen auf, so erzeugt der Korrelator 10 ein Warnsignal und sperrt die weitere Auslösung von Stoßwellen.

## Patentansprüche

1. Verfahren zur Detektion der Güte der Ankopplung des Therapiekopfs (TK) eines Lithotripters zum berührungslosen Zerkleinern von in einem Patientenkörper (PK) sich befindlichen Konkrementen, wobei in dem Therapiekopf (TK) Zertrümmerungswellen erzeugt und über ein Koppelmedium (WK) in den Patientenkörper (PK) eingeleitet werden, dadurch gekennzeichnet, daß mit einem Ultraschallortungsgerät (2), dessen Schallkopf (1) als In-Line-Schallkopf im Therapiekopf (TK) integriert ist, bei angekoppeltem Patientenkörper (PK) mindestens ein Ultraschallbild (X) aufgenommen wird und dieses Ultraschallbild (X) verglichen wird mit einem Ultraschallbild (Y), das bei nicht angekoppelten Patientenkörper (PK) aufgenommen worden ist (Leeraufnahme), wobei die in der Leeraufnahme (Y) auftretenden charakteristischen Wiederholungsechos als Vergleichskriterium dienen.

2. Lithotripter zum berührungslosen Zerkleinern von in einem Patientenkörper (PK) sich befindlichen Konkrementen mit einem Therapiekopf (TK), in dem Zertrümmerungswellen erzeugt und über ein Koppelmedium (WK) in den Patientenkörper (PK) eingeleitet werden und einem Ultraschallortungsgerät (2), dessen Schallkopt (1) als In-Line-Schallkopf im Therapiekopf (TK) integriert ist, gekennzeichnet durch
eine Vorrichtung (15) zur Detektion der Güte der Ankopplung des Therapiekopfs (TK) an den Patientenkörner (PK) mit
- einem Ultraschallbildspeicher (9), in dem ein Ultraschallbild (Y), aufgenommen ohne Ankopplung an den Patientenkörper (Leeraufnahme), gespeichert ist
- einem Multiplizierer (8), in dem ein bei angekoppeltem Patientenkörper (PK) aufgenommenes Ultraschallbild mit den Parametern der Geräteeinstellungen des Ultraschallortungsgeräts (2) (z. B. Greyscale, Zoom, Power) gewichtet wird
- einem Korrelator (10), in dem das bei angekoppeltem Patientenkörper (PK) aufgenommene und mit den Parametern der Geräteeinstellungen gewichteten Ultraschallbild (X) mit der Leeraufnahme (Y) verglichen wird.

3. Lithotripter nach Anspruch 2, gekennzeichnet durch einen vom Korrelator (10) gesteuerten Warnsignalgenerator, der bei der Detektion einer ungenügenden Ankopplung ein Warnsignal erzeugt und/oder den Therapiekopf (TK) abschaltet.
